(19) **Europäisches Patentamt**

**European Patent Office**

**Office européen des brevets**

(11) **EP 1 149 814 B1**

(12) **EUROPÄISCHE PATENTSCHRIFT**

(45) Veröffentlichungstag und Bekanntmachung des Hinweises auf die Patenterteilung:
**07.09.2005 Patentblatt 2005/36**

(51) Int Cl.⁷: **C07C 1/20**, C07C 11/02, C07C 11/09, C07C 11/10

(21) Anmeldenummer: **01106550.5**

(22) Anmeldetag: **15.03.2001**

(54) **Verfahren zur Spaltung von Alkyl-tert.-alkylether zur Gewinnung von Iso-Olefinen und Alkanolen an sauren Katalysatoren**

Process for the acid catalysed decomposition of alkyl tert.alkyl ethers to obtain iso-olefins and alkanols

Procédé de décomposition d'alkyl tertio-alkyl éthers catalysée par un acide pour obtenir des iso-oléfines et des alcools

(84) Benannte Vertragsstaaten:
**AT BE CH CY DE DK ES FI FR GB GR IE IT LI LU MC NL PT SE TR**

(30) Priorität: **28.04.2000 DE 10020943**

(43) Veröffentlichungstag der Anmeldung:
**31.10.2001 Patentblatt 2001/44**

(73) Patentinhaber: **Oxeno Olefinchemie GmbH**
**45764 Marl (DE)**

(72) Erfinder:
• **Sakuth, Michael, Dr.**
  **45770 Marl (DE)**
• **Tuchlenski, Axel, Dr.**
  **45657 Recklinghausen (DE)**
• **Reusch, Dieter, Dr.**
  **45772 Marl (DE)**
• **Beckmann, Andreas, Dr.**
  **45657 Recklinghausen (DE)**

(56) Entgegenhaltungen:
**EP-A- 0 726 241          DE-A- 4 322 712**

• **SCHLEPPINGHOFF ET AL.: "Ultrapure olefins via reaction on specific ion exchange resins" ERDÖL, ERDGAS, KOHLE , Bd. 104, Nr. 4, 1988, Seiten 173-7, XP001007536**
• **DATABASE WPI Section Ch, Week 199809 Derwent Publications Ltd., London, GB; Class A41, AN 1998-099500 XP002171192 & RU 2 083 541 C (NEFTEKHIMSTART CO LTD), 10. Juli 1997 (1997-07-10)**

**Beschreibung**

Die vorliegende Erfindung betrifft ein Verfahren zur Spaltung von Alkyl-tert.-alkylether durch Reaktivdestillation unter saurer Katalyse in die entsprechenden Olefine und Alkanole.

Die Spaltung von Ethern, insbesondere Alkyl-tert.-alkylethem zu Alkanolen und Olefinen ist bekannt und kann zur Herstellung von reinen Olefinen eingesetzt werden. So wird z. B. Isobuten mit technischer Reinheit durch Dehydrierung von $C_4$-Gemischen hergestellt. Diese $C_4$-Gemische enthalten neben Spuren von $C_3$- und $C_5$-Verbindungen Isobuten, 1-Buten und 2-Buten. Eine einfache destillative Trennung dieses Gemisches zur Gewinnung von reinem Isobuten ist aufgrund der sehr geringen Siedepunktdifferenz bzw. Trennfaktor von 1-Buten und Isobuten unwirtschaftlich.

Zur Gewinnung von reinem Isobuten wird daher meist die Rückspaltung von Methyl-tert.-butylether (MTBE) zu Isobuten und Methanol eingesetzt.

Die säurekatalysierte Spaltung von Ethern wie z. B. MTBE zur Gewinnung von reinen Olefinen wie Isobuten ist ein an sich bekanntes Verfahren. Man unterscheidet hierbei zwei unterschiedliche Prozessvarianten. Zum einen kann die Spaltung in der Flüssigphase an sauren Ionentauscherharzen wie beispielsweise in DE 3 509 292 A1 bzw. DE 3 610 704 A1 beschrieben oder an sauren Aluminiumoxiden wie beispielsweise in DD 240 739 A1 offenbart, ausgeführt werden. Im letztgenannten Fall sind die Reaktionsbedingungen (167 °C und 1 bar bzw. 297 °C und 10 bar) so gewählt, dass die MTBE-Spaltung auch im Gas-/Flüssigbereich bzw. in der reinen Gasphase abläuft.

Zum anderen kann die Spaltungsreaktion in der Gas-/Flüssigphase in einer Art kombinierter Reaktionsdestillationskolonne an sauren Katalysatoren durchgeführt werden, so offenbart in EP 0 302 336 A1 oder DE 4 322 712. In EP 0 302 336 A1 wird die Abspaltung von Methanol aus MTBE an einem sauren Ionentauscherharz, das im Kolonnensumpf positioniert ist, beschrieben. Die Spaltung des Ethers findet hier im Kolonnensumpf statt, d. h. der Katalysator wird von einem Gemisch aus Ether, Olefin und Alkohol dauerhaft umspült. Zur Herstellung von Isobuten ist dies nachteilig, da zum einen ein schnelles Abziehen des unter sauren Bedingungen leicht oligomerisierenden Isobutens nicht gewährleistet ist, zum anderen werden die sauren Zentren des Katalysators durch Methanol belegt. In DE 4 322 712 beschreitet man einen anderen Weg. Der tertiäre Ether wird dort oberhalb der Reaktionszone einer Reaktionsdestillationskolonne zugeführt, wobei der Verstärkerteil der Kolonne zur Isobutenreinigung dient, während im Abtriebsteil der Kolonne Methanol vom MTBE-Methanol-Azeotrop abgetrennt wird. Das Azeotrop gelangt zurück in die Reaktionszone. Als sauren Katalysator verwendet man ein sulfatiertes Titandioxid-Extrudat.

Bei beiden Vorgehensweisen können im Zulauf enthaltene Katalysatorgifte, wie beispielsweise Metallionen, den Brönsted-sauren Katalysator desaktivieren. Des Weiteren würde bei dieser Anordnung das Einleiten eines MTBE-Methanol-Gemisches die Reaktionsgeschwindigkeit der MTBE-Spaltung und damit den Umsatz vermindern. Methanol hemmt die eigentliche Spaltungsreaktion aufgrund der Belegung der Säurezentren des Katalysators.

Bei den Spaltungsverfahren, die in der reinen Flüssigphase durchgeführt werden, ist prinzipiell zu beachten, dass keine hohen MTBE-Umsätze erreicht werden können. Dies begründet sich darin, dass es sich bei der Spaltungsreaktion um eine typische Gleichgewichtsreaktion handelt. So setzt sich beispielsweise eine Flüssigphase im Reaktionsgleichgewicht bei 100 °C und entsprechenden Gesamtdruck Folgendermaßen zusammen:

Molenbruch Isobuten = ~ 14 mol-%
Molenbruch MTBE = ~ 70 mol-%
Molenbruch Methanol = ~ 16 mol-%

Ein weiterer Problempunkt bei diesen Verfahren ist das in der homogenen Flüssigphase gelöste Isobuten, das Folgereaktionen eingehen kann. Wichtigste Reaktionen dieser Art sind die säurekatalysierte Dimerisierung und Oligomerisierung. Aus diesem Grunde findet man neben dem gewünschten Zielprodukt Isobuten auch unerwünschte $C_8$- sowie $C_{12}$-Komponenten. Bei den unerwünschten $C_8$-Molekülen handelt es sich um 2,4,4-Trimethyl-1-penten sowie 2,4,4-Trimethyl-2-penten. Des Weiteren findet aufgrund der teils hohen Reaktionstemperaturen eine weitere Folgereaktion statt, bei der zwei Methanolmoleküle unter Abspaltung von Wasser Dimethylether bilden. Da diese Reaktion einen erheblichen Methanolverlust hervorruft, muss vor allem im Hinblick auf eine Kreislaufverschaltung mit einer MTBE-Synthese Frischmethanol zugeführt werden.

Bei derjenigen Verfahrensvariante, bei der man die Spaltungsreaktion in der reinen Gasphase durchführt, treten ebenfalls die Probleme der Dimerisierung bzw. Oligomerisierung des gebildeten Isobutens zu unerwünschten Folgeprodukten auf. Durch Verdünnen des dampfförmigen Eduktstroms mit Inertgas können diese Reaktionen vermindert, aber nicht ausgeschlossen werden. Eine Verdünnung des Eduktstroms senkt gleichzeitig die Effizenz der Produktionsanlage.

Die bei den beschriebenen Verfahren durchgeführten Reaktionen in der Gasphase bzw. bei hohen Temperaturen sind nachteilig, da sich im Verlauf des Spaltprozesses hochsiedende Crackprodukte bilden, die sich auf dem Katalysator ablagern und diesen desaktivieren. Desaktivierte Katalysatoren und/oder hohe Temperaturen begünstigen die Bildung von Nebenprodukten und vermindern die Selektivität der Reaktion. Insbesondere die durch die Etherspaltung erhaltenen Isobutene neigen zur unerwünschten thermischen Polymerisation. Eine Reaktionsführung bei niedrigeren Temperaturen hat häufig einen geringen Umsatz zur Folge.

Der vorliegenden Erfindung lag daher die Aufgabe zugrunde, ein Verfahren zur Spaltung von Alkyl-tert.-alkylethem zu entwickeln, wobei ein hoher Ether-Umsatz bei gleichzeitig geringer Folgeproduktbildung und geringer Katalysatordesaktivierung realisiert werden soll.

Überraschenderweise wurde gefunden, dass die sauer katalysierte Spaltung von Alkyl-tert.-alkylethern zu den entsprechenden Olefinen und Alkanolen bei Einsatz eines Azeotrops aus dem Ether und den enstprechenden Alkanol mit hohen Umsätzen und geringer Nebenproduktbildung durchgeführt werden kann.

Gegenstand der vorliegenden Erfindung ist daher ein Verfahren zur Spaltung von Alkyl-tert.-alkylether in die entsprechenden Iso-Olefine und Alkanole durch sauer katalysierte Reaktivdestillation, wobei die Reaktivdestillationsapparatur in aufsteigender Richtung eine Sumpfzone, mindestens eine Destillationszone und eine Reaktionszone und optional eine weitere Destillationszone aufweist und die Zufuhr des Alkyl-tert.-alkylethers in die Reaktivdestillationsapparatur unterhalb der Reaktionszone zwischen Destillationszone und Reaktionszone durch ein Azeotrop aus dem Alkyl-tert.-alkylether und dem entsprechenden Alkanol erfolgt.

Die Vorteile des erfindungsgemäßen Verfahrens bestehen insbesondere darin, dass bei der Ether-Spaltung durch Reaktivdestillation eine günstige Beeinflussung der endotherm verlaufenden Gleichgewichtsreaktion:

$$\text{Alkyl-tert.-Alkylether} \leftrightarrow \text{Alkanol} + \text{Olefin}$$

durch destillatives Entfemen des Reaktionspartners Olefin resultiert. Weiterhin ist die Olefinkonzentration in der Flüssigphase so gering, dass gegenüber dem reinen Flüssigphasenverfahren die Bildung unerwünschter Folgeprodukte durch Dimerisierung:

$$2 \text{ Olefin} \leftrightarrow \text{Diolefin}$$

bzw. Oligomerisierung vermindert wird.

Als Katalysator im erfindungsgemäßen Verfahren kann ein saures Ionenaustauscherharz oder jeglicher andere saure Katalysator auf anorganischer oder organischer Basis verwendet werden. Der saure Katalysator kann in herkömmlichen Destillations-Reaktionspackungen aus Metallgewebe positioniert werden.

Durch das erfindungsgemäße Verfahren können alle Alkyl-tert.-alkylether gespalten werden, die mit dem entsprechenden Alkanol ein Minimumazeotrop bilden und säurekatalysiert spaltbar sind. Als Edukt für das erfindungsgemäße Verfahren kann daher reiner Alkyl-tert.-alkylether dienen, oder Gemische aus Alkyl-tert.-alkylether mit dem entsprechenden Alkanol und/oder dem Iso-Olefin.

Bei Einsatz des reinen Ethers empfiehlt sich der Zusatz des Alkanols, das durch die Spaltreaktion erzeugt wird. Es ist zwar grundsätzlich möglich, auch andere Alkanole zuzusetzen, jedoch wird die anschließende Aufarbeitung der Alkanole erschwert. Ist die Trennung nicht nötig, d. h. ist das Iso-Olefin das eigentliche Zielprodukt, kann es zur Bildung des Azeotrops hilfreich sein, ein anderes Alkanol als das durch die Spaltung erzeugte zuzusetzen.

Die erfindungsgemäße Spaltung der Alkyl-tert.-alkylether führt zu den entsprechenden Olefinen, d. h. in der Regel zu den verzweigten Olefinen aus dem Molekülteil des Ethers, der die tertiäre Alkylgruppe trägt. Der tertiäre Alkylteil dieser Ether, aus dem nachfolgend das entsprechende Iso-Olefin resultiert, kann 3 bis 10 Kohlenstoffatome enthalten.

Als zweites Spaltprodukt wird das entsprechende Alkanol erhalten. Der Alkylteil der Etheraus dem nachfolgend das entsprechende Alkanol resultiert - kann verzweigt oder unverzweigt sein und 1 bis 10 Kohlenstoffatome enthalten.

Mit dem Verfahren der Erfindung können n-Alkyl-tert.-alkylether in n-Alkanole und Iso-Olefine, sek.-Alkyl-tert.-alkylether in sekundäre Alkanole und Iso-Olefine sowie tert.-Alkyl-tert.-alkylether in tertiäre Alkanole und Iso-Olefine gespalten werden.

Beispiele für solche Verbindungen sind:

| Alkyl-tert.-alkylether | Olefin | Alkanol |
|---|---|---|
| Methyl-tert.-butylether (MTBE) | Isobuten | Methanol |
| Ethyl-tert.-butylether (ETBE) | Isobuten | Ethanol |
| Tert.-amyl-methylether (TAME) | Isoamylen | Methanol |

(fortgesetzt)

| Alkyl-tert.-alkylether | Olefin | Alkanol |
|---|---|---|
| Tert.-amyl-ethylether (TAEE) | Isoamylen | Ethanol |
| tert.-Butyl-isopropylether (TBIPE) | Isobuten | Isopropanol |
| tert.-Butyl-sek.-butylether (TBSBE) | Isobuten | Butanol-2 |
| tert.-Butyl-tert.-butylether (TBTBE) | Isobuten | Isobutanol |

Die Zufuhr des zu spaltenden Alkyl-tert.-alkylethers in die Reaktionszone der Reaktionsdestillationsapparatur erfolgt durch ein Azeotrop aus dem Ether und dem ensprechenden Alkanol.

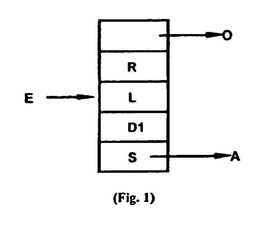

Das Verfahren ist exemplarisch in der Figur 1 dargestellt. Hier bedeuten E: Einspeisung des Edukts, O: Auslass Olefin, A: Auslass Alkanol, R: Reaktionszone, D1: Destillationszone, L: Leerzone (kann auch wegfallen), S: Sumpfzone. Die Sumpfzone wird extern oder intern beheizt, der Olefinauslass mit einem Kondensator versehen (nicht dargestellt).

Das Azeotrop wird erhalten, indem die Zufuhr des Ethers (d. h. des Edukts) in die Reaktivdestillationsapparatur zwischen Destillations- und Reaktionszone erfolgt (z. B. Fig. 1). Als Edukt können Ether/Alkanol-Gemische jeglicher Zusammensetzung verwendet werden. Ein entsprechendes MTBE/Methanol-Gemisch wird häufig durch MTBE-Produktionsanlagen erzeugt.

Nach erfolgter Spaltung des Ether/Alkanol-Azeotrops in der Reaktionszone wird das entsprechende Olefin als Olefin/Alkanol-Azeotrop über den Kopf der Apparatur abgezogen, während der größte Teil des Alkanols in den Sumpf läuft.

Das über Kopf abgezogene Alkanol/Iso-Olefin-Azeotrop kann einen geringen Anteil des Ethers enthalten.

In einer Ausführungsform des Verfahrens weist die Reaktivdestillationsapparatur mehrere Destillationszonen auf, wobei eine Destillationszone in aufsteigender Richtung, (d. h. in Gasströmungsrichtung) über der Reaktionszone angeordnet ist.

In überraschend vorteilhafter Weise ermöglicht die Anordnung der Katalysatorpackung bzw. der Reaktionszone oberhalb des Zulaufes des Edukts, dass Katalysatorgifte wie Metallionen die Reaktionszone nicht erreichen können, sodass eine Desaktivierung des Katalysators auf jeden Fall vermindert wird. Des Weiteren ist eine Auftrennung eines Alkyl-tert.-alkylether/Alkanol-Gemisches bis zum azeotropen Punkt im Verstärkerteil möglich, sodass Alkyl-tert.-alkylether/Alkanol-Gemische jeglicher Zusammensetzung verarbeitet werden können. Es wird so gewährleistet, dass immer eine Ether-haltige Flüssigphase die Reaktionszone erreicht, sodass die Spaltungsreaktion nicht zum Erliegen kommt.

Der geeignete Arbeitsbereich der Reaktivdestillationsapparatur bezüglich des Kolonnendruckes liegt zwischen 1 und maximal 10 bara. Für die Spaltung von MTBE hat sich ein Kolonnendruck von 3-7 bara bewährt. Wird als Katalysator ein z. B. Kationentauscherharz verwendet, ist bei über 125 °C mit einer erheblichen Abspaltung sulfonsaurer Gruppen von der Harzoberfläche zu rechnen, sodass allmählich eine Desaktivierung des Katalysators eintritt. Hier empfiehlt es sich eine Reaktionstemperatur von 105 bis 115 °C.

Über den Kolonnendruck kann die für den Katalysator optimale Betriebstemperatur eingestellt werden.

Andere Katalysatoren, die im erfindungsgemäßen Verfahren verwendet werden können, sind z. B. säureaktivierte Bentonite und/oder Tonerden, Zeolithe, sulfonierte Zirkoniumoxide oder Montmorilonite. Diese Katalysatoren können bei höheren Temperaturen betrieben werden.

Durch die Anordnung der Katalysatorpackung oberhalb des Zulaufs lässt sich die Vergiftung des Katalysators durch Metallionen, die im Feedstrom enthalten sein können, verhindern. Auch können Ether/Alkanol-Gemische beliebiger Zusammensetzung eingesetzt werden, da zwischen Zulauf und Reaktionspackung eine destillative Auftrennung des Gemisches bis zum Azeotrop erfolgen kann. Hierdurch lässt sich auch die Reaktionstemperatur innerhalb der Reaktionszone durch das Verdampfungsgleichgewicht steuern, wodurch eine Schädigung des Katalysators verhindert werden kann. Hier besitzen Verfahren, die mit der Anordnung des Katalysators im Sumpf arbeiteten, einen erheblichen Nachteil, weil Überhitzungen in der Sumpfbeheizung möglich sind, sodass Katalysatorschädigungen auftreten können.

**Patentansprüche**

1. Verfahren zur Spaltung von Alkyl-tert.-alkylether in die entsprechenden Iso-Olefine und Alkanole durch sauer katalysierte Reaktivdestillation,
**dadurch gekennzeichnet,**
**dass** die Reaktivdestillationsapparatur in aufsteigender Richtung eine Sumpfzone, zumindest eine Destillationszone und eine Reaktionszone aufweist, die Zufuhr des Alkyl-tert.-alkylethers in die Reaktivdestillationsapparatur unterhalb der Reaktionszone zwischen Destillationszone und Reaktionszone und die Zufuhr des Alkyl-tert.-alkylethers in die Reaktionszone durch ein Azeotrop aus dem Alkyl-tert.-alkylether und dem entsprechenden Alkanol erfolgt.

**2.** Verfahren nach Anspruch 1,
**dadurch gekennzeichnet,**
**dass** die Reaktivdestillationsapparatur mehrere Destillationszonen aufweist, wobei eine Destillationszone in aufsteigender Richtung über der Reaktionszone angeordnet ist.

**3.** Verfahren nach Anspruch 1 oder 2,
**dadurch gekennzeichnet,**
**dass** n-Alkyl-tert.-alkylether in n-Alkanole und Iso-Olefine gespalten werden.

**4.** Verfahren nach Anspruch 1 oder 2,
**dadurch gekennzeichnet,**
**dass** sek.-Alkyl-tert-alkylether in sekundäre Alkanole und Iso-olefine gespalten werden.

**5.** Verfahren nach Anspruch 1 oder 2,
**dadurch gekennzeichnet,**
**dass** tert.-Alkyl-tert.-alkylether in tertiäre Alkanole und Iso-Olefine gespalten werden.

**6.** Verfahren nach Anspruch 1 oder 2,
**dadurch gekennzeichnet,**
**dass** Methyl-tert.-butylether in Isobuten und Methanol gespalten wird.

**7.** Verfahren nach Anspruch 1 oder 2,
**dadurch gekennzeichnet,**
**dass** Ethyl-tert.-butylether in Isobuten und Ethanol gespalten wird.

**8.** Verfahren nach Anspruch 1 oder 2,
**dadurch gekennzeichnet,**
**dass** Tert-amyl-methylether in Isoamylen und Methanol gespalten wird.

**9.** Verfahren nach Anspruch 1 oder 2,
**dadurch gekennzeichnet,**
**dass** Tert.-amyl-ethylether in Isoamylen und Ethanol gespalten wird.

**10.** Verfahren nach Anspruch 1 oder 2,
**dadurch gekennzeichnet,**
**dass** tert.-Butyl-Isopropylether in Isobuten und Isopropanol gespalten wird.

**11.** Verfahren nach Anspruch 1 oder 2,
**dadurch gekennzeichnet,**
**dass** tert.-Butyl-sek.-butylether in Isobuten und Butanol-2 gespalten wird.

**12.** Verfahren nach Anspruch 1 oder 2,
**dadurch gekennzeichnet,**
**dass** tert.-Butyl-tert.-butylether in Isobuten und Isobutanol gespalten wird.

**Claims**

**1.** A process for the cleavage of an alkyl tert-alkyl ether into the corresponding isoolefin and alkanol by acid-catalysed reactive distillation, **characterized in that** the reactive distillation apparatus has, in an upward direction, a bottom zone, at least one distillation zone and a reaction zone, the alkyl tert-alkyl ether is fed into the reactive distillation apparatus below the reaction zone between a distillation zone and the reaction zone, and the alkyl tert-alkyl ether is fed into the reaction zone by means of an azeotrope of the alkyl tert-alkyl ether and the corresponding alkanol.

**2.** A process according to claim 1, **characterized in that** the reactive distillation apparatus has a plurality of distillation zones, with one distillation zone being located above the reaction zone.

**3.** A process according to either of claims 1 and 2, **characterized in that** an n-alkyl tert-alkyl ether is cleaved into an n-alkanol and an isoolefin.

**4.** A process according to either of claims 1 and 2, **characterized in that** a sec-alkyl tert-alkyl ether is cleaved into a secondary alkanol and an isoolefin.

**5.** A process according to either of claims 1 and 2, **characterized in that** a tert-alkyl tert-alkyl ether is cleaved into a tertiary alkanol and an isoolefin.

**6.** A process according to either of claims 1 and 2, **characterized in that** methyl tert-butyl ether is cleaved into isobutene and methanol.

**7.** A process according to either of claims 1 and 2, **characterized in that** ethyl tert-butyl ether is cleaved into isobutene and ethanol.

**8.** A process according to either of claims 1 and 2, **characterized in that** tert-amyl methyl ether is cleaved into isopentene and methanol.

**9.** A process according to either of claims 1 and 2, **characterized in that** tert-amyl ethyl ether is cleaved into isopentene and ethanol.

**10.** A process according to either of claims 1 and 2, **characterized in that** tert-butyl isopropyl ether is cleaved into isobutene and isopropanol.

**11.** A process according to either of claims 1 and 2, **characterized in that** tert-butyl sec-butyl ether is cleaved into isobutene and 2-butanol.

**12.** A process according to either of claims 1 and 2, **characterized in that** tert-butyl tert-butyl ether is cleaved into isobutene and isobutanol.

**Revendications**

**1.** Procédé de clivage d'éther alkyl-tert-alkylique en iso-oléfines et alcanols correspondants par distillation réactive à catalysation acide,
**caractérisé en ce que**
l'appareillage de distillation réactive présente, dans le sens ascendant, une zone de pot, au moins une zone de distillation et une zone de réaction, l'arrivée de l'éther alkyl-tert-alkylique dans l'appareillage de distillation réactive a lieu au-dessous de la zone de réaction entre la zone de distillation et la zone de réaction, et l'arrivée de l'éther alkyl-tert-alkylique dans la zone de réaction par un azéotrope de l'éther alkyl-tert-alkylique et de l'alcool correspondant.

**2.** Procédé selon la revendication 1,
**caractérisé en ce que**
l'appareillage de distillation réactive présente plusieurs zones de distillation, dont une zone de distillation est disposée, dans le sens ascendant, au-dessus de la zone de réaction.

**3.** Procédé selon la revendication 1 ou 2,
**caractérisé en ce que**
les éthers n-alkyl-tert-alkyliques sont clivés en n-alcanols et en iso-oléfines.

**4.** Procédé selon la revendication 1 ou 2,
**caractérisé en ce que**
les éthers sec-alkyl-tert-alkyliques sont clivés en n-alcanols et iso-oléfines secondaires.

**5.** Procédé selon la revendication 1 ou 2,
**caractérisé en ce que**
les éthers méthyl-tert-butyliques sont clivés en n-alcanols et iso-oléfines tertiaires.

**6.** Procédé selon la revendication 1 ou 2,
**caractérisé en ce que**
les éthers méthyl-tert-butyliques sont clivés en isobutène et en méthanol.

**7.** Procédé selon la revendication 1 ou 2,
**caractérisé en ce que**
les éthers éthyl-tert-butyliques sont clivés en isobutène et en éthanol.

**8.** Procédé selon la revendication 1 ou 2,
**caractérisé en ce que**
les éthers tert-amyl-méthyliques sont clivés en isoamylène et en méthanol.

**9.** Procédé selon la revendication 1 ou 2,
**caractérisé en ce que**
les éthers tert-amyl-éthyliques sont clivés en isoamylène et en éthanol.

**10.** Procédé selon la revendication 1 ou 2,
**caractérisé en ce que**
les éthers tert-butyl-isopropyliques sont clivés en isobutène et en isopropanol.

**11.** Procédé selon la revendication 1 ou 2,
**caractérisé en ce que**
les éthers tert-butyl-sec-butyliques sont clivés en isobutène et en butanol-2.

**12.** Procédé selon la revendication 1 ou 2,
**caractérisé en ce que**
les éthers tert-butyl-tert-butyliques sont clivés en isobutène et en isobutanol.

(Fig. 1)